# EUROPÄISCHE PATENTSCHRIFT

(11) **EP 1 157 064 B1**
(45) Veröffentlichungstag und Bekanntmachung des Hinweises auf die Patenterteilung: **01.09.2004**
(21) Anmeldenummer: 00907548.2
(22) Anmeldetag: 11.02.2000
(51) Int. Cl.: C08K 3/22, A61K 7/48, C01G 9/02

(54) **Verfahren zur Herstellung von NANOPARTIKULÄRE, REDISPERGIERBARE ZINKOXIDGELE**
Process for producing NANOPARTICULATE, REDISPERSIBLE ZINC OXIDE GELS
Procédé pour la préparation de GELS REDISPERSIBLES A BASE DE NANOPARTICULES D'OXYDE DE ZINC REDISPERSIBLES

(30) Priorität: 23.02.1999 DE 19907704
(43) Veröffentlichungstag der Anmeldung: 28.11.2001
(73) Patentinhaber: Bayer Aktiengesellschaft, 51368 Leverkusen (DE)
(72) Erfinder: WOMELSDORF, Hermann-Jens, D-51375 Leverkusen (DE); HOHEISEL, Werner, D-51061 Köln (DE); PASSING, Gerd, D-50937 Köln (DE)
(86) Internationale Anmeldenummer: PCT/EP2000/001116
(87) Internationale Veröffentlichungsnummer: WO 2000/050503

(56) Entgegenhaltungen:
- HILGENDORFF: "from zno colloids to nanocrysalline highly conductive films" J. OF THE ELECTROCHEM. SOCIETY, Bd. 145, Nr. 10, Oktober 1998 (1998-10), Seiten 3632-3637, XP000914529 usa
- PATENT ABSTRACTS OF JAPAN vol. 199, no. 605 & JP 08 026823 A (TOSHIO TSUCHIYA), 30. Januar 1996 (1996-01-30)

## Beschreibung

Die vorliegende Erfindung betrifft ein Verfahren zur Herstellung von nanopartikulären, redispergierbaren Zinkoxidgelen und ihre Verwendung.

Zinkoxid findet für vielfältige Zwecke Verwendung, so z.B. als Weißpigment, als Katalysator, als Bestandteil antibakterieller Hautschutzsalben und als Aktivator für die Kautschukvulkanisation. In Sonnenschutzmitteln und Holzlasuren findet man feinteiliges Zinkoxid als UV-absorbierendes Pigment.

Mit dem Begriff "Nanopartikel" bzw."Nanoteilchen" bezeichnet man allgemein Partikel mit einem Durchmesser von weniger als ca. 100 nm.

Zinkoxidnanoteilchen mit Partikelgrößen unterhalb ca. 30 nm sind potentiell für den Einsatz als UV-Absorber in transparenten organisch-anorganischen Hybridmaterialien, Kunststoffen, Lacken und Beschichtungen geeignet. Daneben ist auch ein Einsatz zum Schutz UV-empfindlicher organischer Pigmente möglich.

Partikel, Partikelaggregate oder -agglomerate aus Zinkoxid, die größer als ca. 30 nm sind, führen zu Streulichteffekten und damit zu einer unerwünschten Abnahme an Transparenz im Bereich des sichtbaren Lichts. Deshalb ist die Redispergierbarkeit, also die Überführbarkeit der hergestellten Zinkoxidnanoteilchen in einen kolloiddispersen Zustand, eine wichtige Voraussetzung für die oben genannten Anwendungen.

Zinkoxidnanoteilchen mit Partikelgrößen unterhalb ca. 5 nm zeigen aufgrund des Größenquantisierungseffektes eine Blauverschiebung der Absorptionskante (L. Brus, J. Phys. Chem. (1986), 90, 2555-2560) und sind daher für den Einsatz als UV-Absorber im UV-A-Bereich weniger geeignet.

Bekannt ist die Herstellung von Zinkoxid durch trockene und nasse Verfahren. Die klassische Methode der Verbrennung von Zink, das trockenes Verfahren (z.B. Gmelin Bd 32, 8. Auflage, Ergänzungsband, S. 772 ff), erzeugt aggregierte Partikel mit einer breiten Größenverteilung. Zwar ist es grundsätzlich möglich, durch Mahlverfahren unter Zuhilfenahme von oberflächenaktiven Agenzien stabile Dispersionen herzustellen, doch aufgrund der zu geringen erzielbaren Scherkräfte sind aus solchen Pulvern Dispersionen mit mittleren Teilchengrößen unterhalb ca. 30 nm nicht erzielbar.

Besonders feinteiliges Zinkoxid wird vor allem nasschemisch durch Fällprozesse hergestellt. Die Fällung in wässriger Lösung liefert in der Regel hydroxid- und/oder carbonathaltige Materialien, die thermisch zu Zinkoxid umgesetzt werden müssen. Die thermische Nachbehandlung wirkt sich dabei auf die Feinteiligkeit negativ aus, da die Partikel dabei Sinterprozessen unterworfen sind, die zur Bildung µm-großer Aggregate führen, die durch Mahlung nur unvollständig auf die Primärpartikel heruntergebrochen werden können.

In JP-A-04 164 814 wird ein Verfahren beschrieben, welches durch Fällung in wässrigem Medium bei erhöhter Temperatur auch ohne thermische Nachbehandlung zu feinteiligem ZnO führt. Als mittlere Teilchengröße wird, ohne Angabe des Agglomerationsgrades, 20 - 50 nm angegeben. Diese Partikel sind verhältnismäßig groß. Dies führt schon bei minimaler Agglomeration zu Streueffekten, die in Transparentanwendungen unerwünscht sind.

In JP-A-07 232 919 wird die Herstellung 5 - 10 000 nm großer ZnO-Partikel aus Zinkverbindungen durch Umsetzung mit organischen Säuren und anderen organischen Verbindungen wie Alkoholen bei erhöhter Temperatur beschrieben. Die Hydrolyse erfolgt hier so, dass die entstehenden Nebenprodukte (Ester der eingesetzten Säuren) abdestilliert werden können. Das Verfahren erlaubt die Herstellung von ZnO-Pulvern, die durch zuvor erfolgte Oberflächenmodifizierung redispergierbar sind. Allerdings ist es auf Basis der Offenbarung dieser Anmeldung nicht möglich, Partikel mit einem mittleren Durchmesser < 15 nm herzustellen. In den in der Anmeldung aufgeführten Beispielen ist dementsprechend als kleinster mittlerer Primärpartikeldurchmesser 15 nm genannt.

In EP 0 893 409 A1 wird die Herstellung von Zinkoxidnanopartikeln wie in JP-A-07 232 919 beschrieben, mit dem Unterschied, dass bei der Fällung des ZnO ein anderes Metalloxid, aus den in der Anmeldung "IIIB" und "IVB" genannten Gruppen des Periodensystems der Elemente, namentlich genannt werden insbesondere Aluminium und Indium, mit gefällt wird.

Weiterhin wurde versucht, durch Hydrolyse von Zinksalzen in Alkoholen direkt zu ZnO zu gelangen (Henglein et al., J. Phys. Chem. 1988, 92, 482-487). Bei Verwendung von NaOH als Base gelang es nicht, stabile und konzentrierte Sole (C_{ZnO} » 10⁻³ Mol/L) zu erhalten.

Durch Hydrolyse von Zinkacetat mit LiOH oder Tetramethylammoniumhydroxid (Spanhel et al., JACS 1991, 113, No.8, 2826-2833) in alkoholischer Lösung wurden konzentrierte Sole erhalten, die neben Zinkoxidnanoteilchen noch Litiumacetat bzw. Tetramethylammoniumacetat in stöchiometrischer Menge enthielten. Damit ist zum einen eine wirtschaftliche Herstellung nicht möglich, da LiOH und Tetramethylammoniumhydroxid vergleichsweise teuer sind, zum anderen ist die Weiterverwendung der Sole stark eingeschränkt, da keine Abtrennung der Nebenprodukte der Fällung erfolgt. Durch Aufkonzentrieren dieser Sole konnten ZnO-haltige Gele erhalten werden, die ebenfalls noch die Nebenprodukte der ZnO-Herstellung in stöchiometrischer Menge enthielten und damit bezüglich ihrer Weiterverwendungsmöglichkeiten stark limitiert waren.

Andere Verfahren zur Herstellung von nanoskaligem Zinkoxid wie das in US-A-5,391,354 beschriebene, das von Zink-Alkoxiden ausgeht, benutzen teure Ausgangsmaterialien und sind daher unwirtschaftlich.

Der vorliegenden Erfindung lag daher die Aufgabe zugrunde, ein nanoskaliges Zinkoxid bereitzustellen, das eine starke UV-Absorption bereits im UV-A-Bereich mit excellenten Dispergiereigenschaften für minimale Streuung vereinigt. Dazu musste ein einfaches Verfahren gefunden werden, das geeignet ist, nanoskaliges Zinkoxid mit einem mittleren Primärpartikeldurchmesser zwischen 5 und 15 nm aus kommerziell verfügbaren, kostengünstigen Edukten in technischem Maßstab preiswert in einer Weise herzustellen, die es erlaubt, das Zinkoxid nach der Herstellung von den Nebenprodukten abzutrennen, ohne dass eine irreversible Aggregation der Partikel auftritt und nach Redispergierung ohne aufwendige Mahlung in Form von Solen zur weiteren Verwendung bereitzustellen.

Ausgehend von der von Henglein et al. beschriebenen Methode zur Hydrolyse von Zinkacetat in alkoholischen Medien wurde nun überraschend gefunden, dass durch Hydrolyse von Zinkverbindungen mit Basen in Alkohol- oder Alkohol-Wasser-Gemischen nach Abtrennung der überstehenden, mit den Nebenprodukten der Fällung beladenen Lösung, Zinkoxidgele enthaltend Zinkoxidnanoteilchen mit mittleren Primärpartikeldurchmessern ≤ 15 nm erhalten werden können, die einfach durch Zugabe geeigneter Lösemittel oder Lösemittelkombinationen - gegebenenfalls zusammen mit geeigneten Oberflächenmodifikatoren - und Verrühren, unter Verzicht auf Mahlschritte oder andere aufwendige mechanische Zerkleinerungsverfahren, vollständig zu weitgehend primärpartikeldispersen Zinkoxid-Solen redispergiert werden können, ohne dass ein signifikanter Qualitätsverlust bezüglich der Monodispersität und Größe der Teilchen auftritt.

Gegenstand der Erfindung ist daher ein Verfahren zur Herstellung von nanopartilkulären, redispergierbaren Zinkoxidgelen, enthaltend Zinkoxidpartikel mit einem durch eine transmissionselektronenmikroskopische Aufnahme bestimmten mittleren kreisäquivalenten Primärpartikeldurchmesser von ≤ 15 nm, durch basische Hydrolyse mindestens einer Zinkverbindung in Alkohol oder einem Alkohol-Wasser-Gemisch, dadurch gekennzeichnet, dass man die bei der Hydrolyse zunächst entstehende Fällung reifen lässt, bis das Zinkoxid komplett ausgeflockt ist, diese Fällung dann zu einem Gel verdichtet und von der überstehenden Phase abtrennt.

Unter mittlerem Primärpartikeldurchmesser im Sinne der Erfindung wird der mittlere kreisäquivalente Primärpartikeldurchmesser der Zinkoxidpartikel verstanden, der in einer transmissionselektronenmikroskopischen Aufnahme bestimmt werden kann.

Das erfindungsgemäße Verfahren wird vorzugsweise so durchgeführt, dass man mindestens eine Zinkverbindung in Alkohol oder einem Alkohol-Wasser-Gemisch ganz oder teilweise löst und durch Zugabe mindestens einer Base bei Temperaturen zwischen dem Gefrierpunkt der Lösung und deren Siedepunkt hydrolysiert. Dabei entsteht, in Abhängigkeit von Temperatur und Konzentration, gegebenenfalls mit zeitlicher Verzögerung, eine weißliche Fällung.

Die Fällung kann, sobald kein oder fast kein Zinkoxid mehr kolloidal dispers vorliegt, gegebenenfalls nach zwischengeschalteten Waschschritten, bei denen nach dem Absetzen der Fällung der Überstand gegen frisches Lösemittel ausgetauscht wird, zu einem Gel verdichtet und von der überstehenden Phase abgetrennt werden. Die Prüfung, ob kein Zinkoxid mehr kolloidal dispers vorliegt, kann durch Filtrierung einer Probe des Reaktionsansatzes über einen Filter mit 0,2 µm Porengröße und Prüfung des Filtrats auf Tyndall-Effekt geschehen. Die Verdichtung der Fällung zum Gel kann durch Absetzen und Stehenlassen oder durch Zentrifugation erfolgen, bevorzugt wird die Fällung zentrifugiert.

Das so erhaltene Zinkoxidgel kann durch Zugabe von organischen Lösemitteln und/oder Wasser, gegebenenfalls unter Zugabe von oberflächenmodifizierenden Verbindungen, zu weitgehend primärpartikeldispersen Solen redispergiert werden. Dabei wird das Redispergieren bevorzugt durch Zumischen des entsprechenden Lösungsmittels oder der Lösungsmittelgemische unter Rühren durchgeführt.

Bei der in dem erfindungsgemäßen Verfahren eingesetzten Zinkverbindung handelt es sich vorzugsweise um salzartige Verbindungen, die in dem gewählten Alkohol oder Wasser-Alkohol-Gemisch ganz oder teilweise löslich sind, besonders bevorzugt ist Zinkacetat, ganz besonders bevorzugt dessen Dihydrat.

Als Alkohol werden bevorzugt Monoalkohole eingesetzt, ganz besonders bevorzugt Methanol.

Als Base können OH- oder NH-Gruppen-haltige basische Verbindungen eingesetzt werden, besonders solche, die in konzentrierter Form erhältlich und in Alkohol oder Alkohol-Wasser-Gemischen löslich sind. Dazu gehören insbesondere Natrium- und Kaliumhydroxid und Aminbasen, wobei erstere bevorzugt sind. Die Base kann sowohl in fester Form, beispielsweise als NaOH oder KOH-Plätzchen oder in gelöster Form im erfindungsgemäßen Verfahren eingesetzt werden. In einer bevorzugten Ausführungsform der Erfindung wird die Base in gelöster Form zur Lösung des Zinksalzes gegeben. Dabei wird die Base bevorzugt in Alkohol, Wasser oder Alkohol-Wasser-Gemischen gelöst, besonders bevorzugt in Methanol, Wasser oder Methanol-Wasser-Gemischen.

Die Hydrolyse wird bevorzugt mit nichtstöchiometrischen Mengen Base durchgeführt, besonders bevorzugt unterstöchiometrisch, ganz besonders bevorzugt mit einem Zn zu OH Verhältnis von 1 zu 1,6 bis 1 zu 1,95.

Der minimale Wassergehalt des Reaktionsansatzes wird durch den Wassergehalt der eingesetzten Edukte und durch die Menge des entstehenden Zinkoxids bestimmt. Wasser kann darüberhinaus zugesetzt werden, um besondere Effekte zu erzielen, z.B. eine Beschleunigung der ZnO-Bildung oder eine bessere Löslichkeit der Edukte.

In einer bevorzugten Ausführungsform der Erfindung wird mehr Zinksalz eingesetzt, als es dem Löslichkeitsprodukt in dem verwendeten Lösemittel entspricht. Durch Zugabe eines Bruchteils der für die Hydrolyse vorgesehenen Menge Base wird die Löslichkeit der Zinksalze verbessert, ohne dass bereits Zinkoxid entsteht.

In einer weiteren bevorzugten Ausführungsform der Erfindung werden das Lösen der Edukte und die Hydrolyse unter Inertgasspülung durchgeführt.

In einer weiteren bevorzugten Ausführungsform wird als Zinkverbindung ein handelsübliches, grobteiliges Zinkoxid eingesetzt, das in einer vorgeschalteten Reaktion zu einer geeigneten Zinkverbindung, vorzugsweise Zinkacetat, umgesetzt wird. In einer besonders bevorzugten Ausführungsform der Erfindung geschieht dies durch Umsetzung von Zinkoxid mit Eisessig oder mit einem Eisessig/Acetanhydrid-Gemisch in einem Alkohol-Wasser-Gemisch.

In einer bevorzugten Ausführungsform der Erfindung liegt die Temperatur beim Lösen und Umsetzen zwischen 0°C und dem Siedepunkt der eingesetzten Lösemittel. In einer besonders bevorzugten Ausführungsform der Erfindung wird die Temperatur vor und/oder während und/oder nach der Fällung bis zum Siedepunkt des Gemisches oder einer Temperatur darunter erhöht.

Zur Beeinflussung der Morphologie und/oder der Kristallinität der Zinkoxidpartikel kann vor, während oder nach der Fällung ein Zusatz von geeigneten Verbindungen (Fremdionen) vorgenommen werden. Bevorzugt sind Verbindungen der 2.-4. Hauptgruppe und Übergangsmetallverbindungen. Besonders bevorzugt sind Mangan-, Magnesium-, Silizium- und Aluminiumverbindungen, ganz besonders bevorzugt Aluminium- und Siliciumalkoxide, Aluminate und Silikate. In einer bevorzugten Ausführungsform des erfindungsgemäßen Verfahrens werden diese Verbindungen in gelöster Form zum Reaktionsansatz gegeben. Dabei werden bevorzugt 0,01-3 Mol%, bezogen auf Zink, verwendet.

Die Fällung kann beispielsweise durch Dekantieren oder Absaugen des Überstandes isoliert werden. Dabei ist es günstig, die Fällung gut zu verdichten, beispielsweise durch lange Absetzzeiten, da das Redispergierverhalten der Fällung vom Salz- und Lösemittelgehalt abhängt. Bei längerem Absetzen verdichtet sich die Fällung zu einem hochviskosen Gel. Eine besonders hohe Verdichtung des Materials und damit auch eine besonders vollständige Abtrennung des Zinkoxids von den Nebenprodukten der Fällung wird durch Zentrifugation erzielt. Dabei wird ein transluzentes, festes Gel mit hohem Feststoffanteil erhalten, welches besonders gut redispergierbar ist.

In einer weiteren bevorzugten Ausführungsform wird der Salzgehalt der Fällung nach Absetzen durch Absaugen eines Teils des Überstandes und Zugabe von frischem Lösemittel verringert. Besonders bevorzugt wird der Salzgehalt der Fällung dann durch Absaugen eines Teils des Überstandes und Zugabe von frischem Lösemittel verringert, wenn das entstehende Zinkoxidgel in Wasser oder Alkohol-Wasser-Gemischen, insbesondere Diol- und/oder Polyol-Wasser-Gemischen, bevorzugt unter Verwendung von oberflächenmodifizierenden Verbindungen zu einem Sol redispergiert werden soll.

Das so erhaltene Zinkoxidgel kann durch geeignete Maßnahmen in ein kolloiddisperses Sol überführt werden. In einer bevorzugten Ausführungsform der Erfindung geschieht dies durch Zugabe von organischen Lösemitteln, bevorzugt polaren aprotischen Lösemitteln, ganz besonders bevorzugt Dichlormethan und/oder Chloroform. In einer weiteren bevorzugten Ausführungsform wird das Gel in Wasser redispergiert. In einer weiteren bevorzugten Ausführungsform wird das Gel in Alkohol-Wasser-Gemischen, insbesondere Diol- und/oder Polyol-Wasser-Gemischen, bevorzugt unter Verwendung von oberflächenmodifizierenden Verbindungen redispergiert. Als oberflächenmodifizierende Verbindungen sind stickstoffhaltige Verbindungen bevorzugt, besonders bevorzugt ist Triethanolamin.

Mahlschritte oder andere aufwendige mechanische Zerkleinerungsschritte sind nicht notwendig.

Die Zugabe erfolgt bevorzugt in einem Masseverhältnis Zinkoxidgel zu Lösemittel von 1 : 0,4 bis 1 : 10, besonders bevorzugt im Verhältnis 1 : 0,4 bis 1 : 3, ganz besonders bevorzugt im Verhältnis 1 : 0,7 bis 1 : 1,5. Die Massenverhältnisse, die benötigt werden, um ein stabiles Sol zu erhalten, variieren je nach verwendetem Lösemittel.

In einer weiteren bevorzugten Ausführungsform kann die Fällung vor dem Verdichten zum Gel durch Zugabe von Dichlormethan und/oder Chloroform in ein Zinkoxid-Sol überführt werden, in dem die Zinkoxidpartikel kolloiddispers vorliegen. Dies geschieht bevorzugt nach vorheriger Verringerung des Salzgehaltes der Fällung durch wiederholtes Absetzen und Absaugen eines Teils des Überstandes und Zugabe von frischem Lösungsmittel.

Ein weiterer Gegenstand der Erfindung ist die Verwendung des erfindungsgemäßen Zinkoxidgels oder der daraus hergestellten Sole in organisch-anorganischen Hybridmaterialien, insbesondere für den UV-Schutz von polymeren Werkstoffen, Lacken und Beschichtungen, insbesondere für Transparentanwendungen. Daneben ist auch ein Einsatz zum Schutz UV-empfindlicher organischer Pigmente und Farbstoffe möglich. Daneben sind die erfindungsgemäßen Zinkoxidgele und Zinkoxidsole auch zur Matrixmodifizierung von Polymeren, Lacken und Beschichtungen und als verbesserter Vulkanisationsaktivator für Kautschuke und Latices geeignet.

### Beispiele

Die Erfindung wird anhand der folgenden Beispiele weiter erläutert, ohne dabei auf diese Beispiele beschränkt zu sein.

Die Zentrifugationsschritte wurden in einer Laborzentrifuge der Fa. Heraeus (Variofuge RF) mit einem Rotor mit 20,4 cm Rotorradius durchgeführt.

### Beispiel 1

590 g Zinkacetat-Dihydrat wurden in einem 6L-Kolben in 2000 g Methanol bei 55°C gelöst und mit einer auf Raumtemperatur temperierten Lösung von 302 g Kaliumhydroxidplätzchen (84,7 %) in 1000 g Methanol unter Rühren versetzt. Es entstand sofort eine weiße, voluminöse Fällung, die man 14 h absetzen liess. Anschließend wurden vom Überstand 3165 g abgesaugt und durch 1000 g Methanol ersetzt. Es wurde nun etwa 20 min gerührt. Nach 75 min Absetzzeit wurden weitere 806 g Überstand abgesaugt und durch 500 g Methanol ersetzt. Es wurde noch 40 min gerührt, dann nach 40 min Absetzzeit erneut 786 g Überstand abgesaugt und durch 500 g Methanol ersetzt. Nach 30 min Rührzeit wurde zentrifugiert (5500 Umdrehungen/min, 30 min). Nach der Zentrifugation betrug die Masse des erhaltenen Zinkoxidgels 251 g.

Der Feststoffgehalt des Gels betrug 75,3 % (Trocknung: 1h bei 130°C). Eine Elementaranalyse ergab einen Zinkgehalt von 75,7 %, entsprechend 93,9 % ZnO. Daneben wurden 0,25 % Kalium, entsprechend ca. 0,63 % Kaliumacetat gefunden.

Ein Röntgendiffraktogramm der getrockneten Probe zeigte ausschließlich hexagonales Zinkoxid. Eine Auswertung der Reflexe nach Scherrer ergab eine mittlere Kristallitgröße von 6,9 nm (Reflexe: 100 und 002, 100 doppelt gewichtet, da im hexagonalen Kristallsystem identisch mit 010).

### Beispiel 2

In einen 6 Liter-Vierhalskolben wurden 218,5 g Zinkoxid (99,8 %) vorgelegt und mit 1200 g Methanol, 330g Eisessig und 46,5 g deionisiertem Wasser gemischt und unter Rühren auf 60°C erhitzt. Parallel dazu wurde eine Lösung aus 301,68 g Kaliumhydroxidplätzchen (84,7 %) und 700 g Methanol unter Kühlung angesetzt. Nachdem das im Kolben vorliegende Gemisch 60°C erreicht hatte, wurden ca. 80 ml der angesetzten KOH-Methanol-Lösung über einen Tropftrichter langsam hinzudosiert. Die Mischung klarte langsam auf und war nach ca. 30 min, klar. Nun wurde bei 60°C der Rest der KOH-Methanol-Lösung innerhalb 30 Sekunden über einen Tropftrichter zudosiert. Es entstand sofort eine weiße starke Trübung, die Temperatur der Mischung stieg um ca. 2°C an. Nach einigen Minuten klarte die Mischung etwas auf und wurde anschließend wieder milchig weiß. Nun wurde die Heizquelle entfernt und die Mischung in einem Eisbad 20 min. abgekühlt. Der Rührer wurde bei einer Temperatur von 16°C abgestellt. Nach ca. 2 Stunden befand sich im Kolben ein weißer Bodensatz, der Überstand war fast klar. Vom Überstand wurden 2078 g abgesaugt und durch 750 g Methanol ersetzt. Es wurde nun ca. 60 min, gerührt und anschließend wurde der Reaktionsansatz im Verhältnis 1:1 geteilt. Eine Hälfte (A) wurde wie folgt weiterverarbeitet: Nach 14 h Absetzen wurde der Überstand abdekantiert und durch 375 g Methanol ersetzt. Es wurde nun ca. 50 min gerührt und dann nach erneutem Absetzen und Abdekantieren wiederum mit 375 g Methanol versetzt. Es wurde nun wiederum ca. 50 min gerührt, anschließend bei 5500 U/min⁻¹ 30 min zentrifugiert und der Überstand wurde abdekantiert. Das Gelgewicht betrug 130,6 g.

Der Feststoffgehalt des Gels betrug 75,8 % (Trocknung: 1h bei 130°C). Eine Elementaranalyse ergab einen Zinkgehalt von 76 %, entsprechend 94,6 % ZnO. Daneben wurden 0,09 % Kalium, entsprechend ca. 0,21 % Kaliumacetat gefunden.

Ein Röntgendiffraktogramm der getrockneten Probe zeigte ausschließlich hexagonales Zinkoxid. Eine Auswertung der Reflexe nach Scherrer ergab eine mittlere Kristallitgröße von 7,9 nm (Reflexe: 100 und 002, 100 doppelt gewichtet, da im hexagonalen Kristallsystem identisch mit 010).

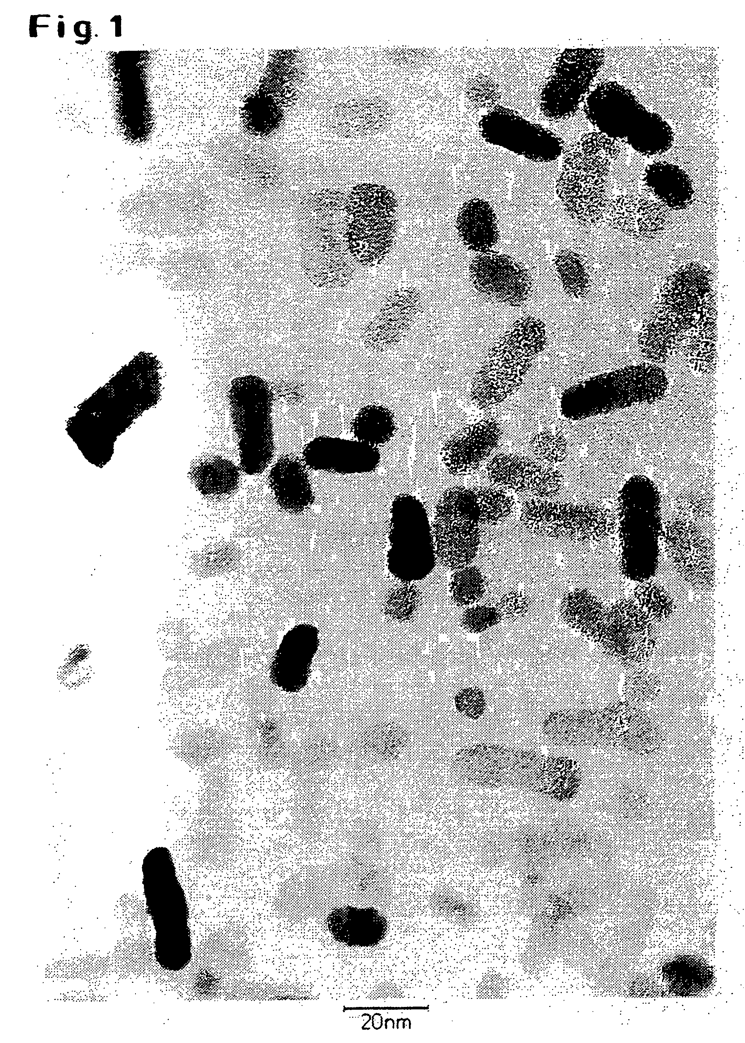

Eine transmissionselektronenmikroskopische Aufnahme einer in einem Ethylenglykol-Wasser-Gemisch verdünnten Probe des Gels (A) zeigt Abbildung 1.

Die andere Hälfte des Reaktionsansatzes wurde durch Zentrifugation bei 5500 min⁻¹, 30 min zum Gel verdichtet. Das Gelgewicht des Gels (B) betrug 134,1 g.

### Beispiel 3

In einen 6 Liter-Vierhalskolben wurden 218,5 g Zinkoxid (99,8%) vorgelegt und mit 1200 g Methanol, 328,5 g Eisessig und 46,5 g deionisiertem Wasser gemischt und unter Rühren auf 60°C erhitzt. Parallel dazu wurden eine Lösung aus 308,1 g Kaliumhydroxidplätzchen (84,7 %) und 700 g Methanol unter Kühlung angesetzt. Nachdem das Gemisch im Kolben 60°C erreicht hatte, wurden ca. 90 ml der angesetzten KOH-Methanol-Lösung über einen Tropftrichter langsam hinzudosiert. Die Lösung klarte langsam auf und war nach ca. 15 min. klar. Nun wurde eine Lösung von 2,8 g Aluminium-tri-sek.-isobutylat in 20 g 2-Propanol hinzugegeben. Dann wurde der Rest der KOH-Methanol-Lösung innerhalb 30 Sekunden über einen Tropftrichter zudosiert. Es entstand sofort eine weiße starke Trübung, die Temperatur der Mischung stieg um ca. 2°C an. Nach einigen Minuten klarte die Mischung etwas auf und wurde anschließend wieder milchig weiß. Nun wurde die Heizquelle entfernt und die Mischung in einem Eisbad abgekühlt. Der Rührer wurde bei einer Temp. von 23°C abgestellt. Nach 14 h Absetzzeit hatte sich ein weißer Bodensatz gebildet. Vom Überstand wurden 1920,7 g abgesaugt und durch 700 g Methanol ersetzt. Es wurde nun ca. 45 min. gerührt und anschließend wurde die Mischung im Verhältnis 1:1 geteilt. Eine Hälfte wurde wie folgt weiterverarbeitet: Nach 3 Stunden Absetzzeit wurde der Überstand abdekantiert und durch 300 g Methanol ersetzt. Es wurde nun ca. 45 min gerührt. Nach erneutem 14-stündigem Absetzen und Abdekantieren wurde wiederum mit 300 g Methanol versetzt. Es wurde wiederum für ca. 60 min gerührt.

Anschließend wurde bei 5500 U/min⁻¹ 30 min zentrifugiert und der Überstand wurde abdekantiert. Das Gelgewicht betrug 133 g.

Das Feststoffgehalt des Gels betrug 78,7 % (Trocknung: 1h bei 130 °C). Eine Elementaranalyse ergab einen Zinkgehalt von 72 %, entsprechend 89,6 % ZnO. Daneben wurden 0,17 % Kalium, entsprechend ca. 0,43 % Kaliumacetat und 0,15 % Aluminium, entsprechend 0,31 % Al₂O₃ gefunden.

Ein Röntgendiffraktogramm der getrockneten Probe zeigte ausschließlich hexagonales Zinkoxid. Eine Auswertung der Reflexe nach Scherrer ergab eine mittlere Kristallitgröße von 7,7 nm (Reflexe: 100 und 002, 100 doppelt gewichtet, da im hexagonalen Kristallsystem identisch mit 010).

### Beispiel 4

In einem 2 L Planschliffgefäß mit mechanischem Rührer, Kühler, Thermometer und Argonbeschleierung wurden 770 ml Methanol vorgelegt und auf 40°C erhitzt. Dann wurden 284,91 g Zinkacetat-Dihydrat zudosiert und gelöst und die Lösung anschließend auf 60°C erwärmt. Die Lösung war etwas trübe. Nach Zugabe von 4,60 g Natriumhydroxidplätzchen (98,8 %-ig) wurde eine klare Lösung erhalten. Anschließend wurden in 7 Minuten 173,29 g wässrige Natriumhydroxid-Lösung (49,8 %-ig) zugegeben, wobei die Temperatur auf maximal 64°C anstieg. Dabei trübte die Lösung sofort ein und es bildete sich eine weiße Fällung, die eine Stunde bei 60°C gerührt wurde. Danach wurde auf Raumtemperatur abgekühlt. Das Volumen der Mischung betrug 960 ml. Nach einer Absetzzeit von 14 h wurde der trübe Überstand abgesogen, zum Bodensatz wurden 750 ml Methanol gegeben und es wurde 30 Minuten gerührt. Nach 4 Stunden hatte die Fällung erneut abgesetzt, der klare Überstand wurde abgesogen, zum Bodensatz wurden 750 ml Methanol gegeben und es wurde 30 Minuten gerührt. Nach einer erneuten Absetzzeit von 14 h und dem Absaugen des klaren Überstandes wurden zum Bodensatz 250 ml Methanol gegeben und es wurde 10 Minuten bei 5500 U/min⁻¹ zentrifugiert. Es wurden 129,14 g Gel erhalten.

Der Feststoffgehalt des Gels betrug 79,1 % (Trockenrückstand: 1h, 130°C). Die Elementaranalyse des Trockenrückstandes ergab einen Zinkgehalt von 77,0 %, entsprechend 95,8 % ZnO. Der Natriumgehalt betrug 0,22 %, entsprechend 0,78 % Natriumacetat. Das Röntgendiagramm des Gel-Trockenrückstandes zeigte ausschließlich hexagonales ZnO. Die Auswertung der Reflexe nach Scherrer ergab eine mittlere Kristallitgröße von 9,4 nm.

### Beispiel 5

In einem 2 L Planschliffgefäß mit mechanischem Rührer, Kühler, Thermometer und Argonbeschleierung wurden 770 ml Methanol vorgelegt, darin 122,30 g Zinkoxid (99,8%) eingetragen und auf 50 °C erhitzt. Dann wurde eine Lösung aus 183 g Eisessig (100 %-ig) und 27 g Wasser in 10 Minuten zudosiert und die Mischung anschließend auf 60°C erwärmt. Es entstand eine leicht trübe Lösung. Nach der Zugabe von 7,56 g Natriumhydroxidplätzchen (98,8 %-ig) wurde die Lösung wasserklar. Anschließend wurde auf 21°C abgekühlt und in 60 Sekunden 200,91 g wässrige Natriumhydroxid-Lösung (49,5 %-ig) zugegeben, wobei die Temperatur kurz auf 51°C anstieg. Dabei trübte die Lösung sofort ein und es bildete sich eine weiße Fällung, die eine Stunde bei 60°C gerührt wurde. Anschließend wurde in Eiswasser auf Raumtemperatur abgekühlt. Das Volumen der Mischung betrug 1100 ml. Die Mischung wurde 30 Minuten bei 500 min⁻¹ zentrifugiert, ohne dass sich hierbei die Fällung zu einem Gel verdichtete. Der Überstand wurde abgesogen, der Bodensatz wurde mit 750 ml Methanol durch 15 min Rühren resuspendiert. Es wurde erneut 45 min bei 500 min⁻¹ zentrifugiert, der klare Überstand wurde abgesogen und der Bodensatz wieder mit 750 ml Methanol 30 Minuten resuspendiert. Nach erneuter 45 minütiger Zentrifugation bei 500 min⁻¹ und dem Absaugen des klaren Überstandes wurde der Bodensatz mit 250 ml Methanol resuspendiert und durch 10 minütige Zentrifugation bei 5200 min⁻¹ zum Gel verdichtet. Es wurden 168,5 g Gel erhalten.

Der Feststoffgehalt des Gels betrug 74,1 % (1 h, 130°C). Die Elementaranalyse ergab einen Zinkgehalt von 78 %, entsprechend 97,1 % ZnO. Der Natriumgehalt betrug 0,87 %, entsprechend 3,1 % Natriumacetat. Das Röntgendiagramm des Gel-Trockenrückstandes zeigte ausschließlich hexagonales ZnO. Die Auswertung der Reflexe nach Scherrer ergab eine mittlere Kristallitgröße von 10,2 nm.

### Beispiel 6

### Herstellung eines Zinkoxid-Sols

Zu 134,1 g des in Beispiel 2 hergestellten Zinkoxidgels (B) wurden 134,1 g Dichlormethan hinzugegeben und gelegentlich geschüttelt. Es entstand ein transluzentes Sol (244,4 g). Die Elementaranalyse ergab einen Zinkgehalt von 31 %, entsprechend 38,6 % ZnO. Durch Auswertung von transmissionselektronenmikroskopischen Aufnahmen einer in einem Ethylenglykol-Wasser-Gemisch verdünnten Probe des Sols (Fig.1, Vergrößerung 1000000 : 1) wurde der mittlere, kreisäquivalente Primärpartikeldurchmesser der Zinkoxidpartikel zu 13 nm (Zahlenmittel aus 283 ausgezählten Partikeln) bestimmt.

### Beispiel 7

### Herstellung eines Zinkoxid-Sols

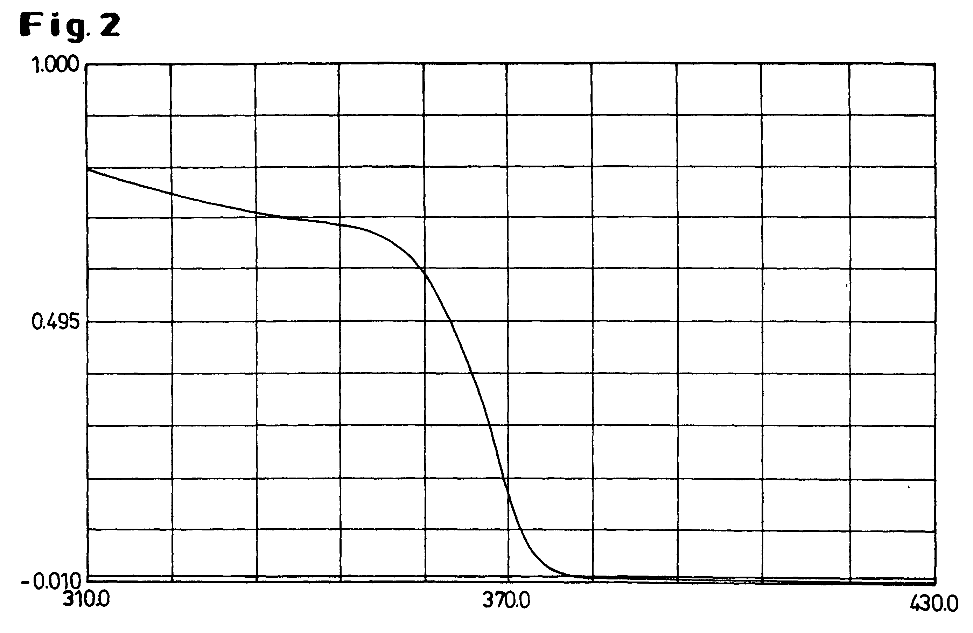

300 g eines analog Beispiel 1 hergestellten Zinkoxidgels wurden mit 291 g Ethylenglykol, 145 g Wasser und 82 g Triethanolamin verrührt. Der Methanolanteil des Gels wurde bei Raumtemperatur im Vakuum abgezogen. Es wurden 683 g eines gelblich transluzenten Sols erhalten. Durch Auswertung von transmissionselektronenmikroskopischcn Aufnahmen einer in einem Ethylenglykol-Wasser-Gemisch verdünnten Probe des Sols wurde der mittlere, kreisäquivalente Primärpartikeldurchmesser der Zinkoxidpartikel zu 10,2 nm (Zahlenmittel aus 584 ausgezählten Partikeln) bestimmt. Das optische Absorptionsspektrum einer in einem Ethylenglykol-Wasser-Gemisch verdünnten Probe dieses Sols zeigt Fig. 2 (1: Extinktion, 2: Wellenlänge [nm]).

Nach 3 Monaten Standzeit war das Sol äußerlich unverändert. Eine erneute Bestimmung des mittleren, kreisäquivalenten Primärpartikeldurchmessers der Zinkoxidpartikel durch Auswertung einer transmissionselektronenmikroskopischen Aufnahmen einer in einem Ethylenglykol-Wasser-Gemisch verdünnten Probe des Sols ergab einen Wert von 9,4 nm (Zahlenmittel aus 803 ausgezählten Partikeln).

## Patentansprüche

1. Verfahren zur Herstellung von nanopartikulären, redispergierbaren Zinkoxidgelen, enthaltend Zinkoxidpartikel mit einem durch eine transmissionselektronenmikroskopische Aufnahme bestimmten mittleren kreisäquivalenten Primärpartikeldurchmesser von ≤ 15 nm, durch basische Hydrolyse mindestens einer Zinkverbindung in Alkohol oder einem Alkohol-Wasser-Gemisch, **dadurch gekennzeichnet, dass** man die bei der Hydrolyse zunächst entstehende Fällung reifen lässt, bis das Zinkoxid komplett ausgeflockt ist, diese Fällung dann zu einem Gel verdichtet und von der überstehenden Phase abtrennt.

2. Verfahren zur Herstellung von Zinkoxidgelen nach Anspruch 1, **dadurch gekennzeichnet, dass** vor, während oder nach der Fällung 0,01 bis 3 Mol-% Fremdionen, bezogen auf den Anteil an Zink, zugesetzt werden.

3. Verfahren nach einem oder mehreren der Ansprüche 1 und 2, **dadurch gekennzeichnet, dass** als Zinkverbindung Zinkacetat und/oder Zinkacetat-Dihydrat verwendet wird.

4. Verfahren gemäß Anspruch 3, **dadurch gekennzeichnet, dass** das Zinkacetat und/oder Zinkacetat-Dihydrat in einem vorgeschalteten Verfahren aus Zinkoxid hergestellt wird.

5. Verfahren gemäß einem oder mehreren der vorangegangenen Ansprüche 1 bis 4, **dadurch gekennzeichnet, dass** die Hydrolyse mit unterstöchiometrischen Mengen an Base bezogen auf die Zinkverbindung durchgeführt wird.

6. Verfahren zur Herstellung von Zinkoxidsolen, **dadurch gekennzeichnet, dass** Zinkoxidgele nach Anspruch 1 in organischen Lösemitteln und/oder Wasser, gegebenenfalls unter Zusatz von oberflächenmodifizierenden Verbindungen, redispergiert werden.

7. Verfahren zur Herstellung von Zinkoxidsolen nach Anspruch 6 durch basische Hydrolyse mindestens einer Zinkverbindung in Alkohol oder einem Alkohol-Wasser-Gemisch, **dadurch gekennzeichnet, dass** man die bei der Hydrolyse zunächst entstehende Fällung reifen lässt, bis das Zinkoxid komplett ausgeflockt ist und diese Fällung durch Zugabe von Dichlormethan und/oder Chloroform redispergiert.

8. Verwendung von Zinkoxidgelen, die nach einem Verfahren gemäß einem der Ansprüche 1 bis 5 hergestellt wurden, für die Matrixmodifizierung von Polymeren, Lacken und Beschichtungen, als verbesserter Vulkanisationsaktivator für Kautschuke und Latices, für den UV-Schutz von Polymeren, Lacken und Beschichtungen sowie für den UV-Schutz empfindlicher organischer Farbstoffe und Pigmente.

9. Verwendung von Zinkoxidsolen, die nach einem Verfahren gemäß einem der Ansprüche 6 oder 7 hergestellt wurden, für die Matrixmodifizierung von Polymeren, Lacken und Beschichtungen, als verbesserter Vulkanisationsaktivator für Kautschuke und Latices, für den UV-Schutz von Polymeren, Lacken und Beschichtungen sowie für den UV-Schutz empfindlicher organischer Farbstoffe und Pigmente.

10. Zinkoxidgele, **dadurch gekennzeichnet, dass** sie nach einem Verfahren gemäß einem der Ansprüche 1 bis 5 hergestellt wurden.

11. Zinkoxidsole, **dadurch gekennzeichnet, dass** sie nach einem Verfahren gemäß einem der Ansprüche 6 oder 7 hergestellt wurden.

## Claims

1. Process for producing nanoparticulate, redispersible zinc oxide gels, containing zinc oxide particles having an average circular equivalent primary particle diameter, determined from a transmission electron micrograph, of ≤ 15 nm, by basic hydrolysis of at least one zinc compound in alcohol or a mixture of alcohol and water, **characterised in that** the precipitate that is initially produced during the hydrolysis is allowed to mature until the zinc oxide has completely flocculated, this precipitate then being compressed to form a gel and separated from the supernatant phase.

2. Process for producing zinc oxide gels according to claim 1, **characterised in that** 0.01 to 3 mol % of foreign ions, based on the proportion of zinc, are added before, during or after precipitation.

3. Process according to either or both of claims 1 and 2, **characterised in that** the zinc compound used is zinc acetate and/or zinc acetate dihydrate.

4. Process according to claim 3, **characterised in that** the zinc acetate and/or zinc acetate dihydrate is/are produced from zinc oxide in a prior process.

5. Process according to any one or more of claims 1 to 4, **characterised in that** the hydrolysis is carried out with substoichiometric amounts of base, based on the zinc compound.

6. Process for producing zinc oxide sols, **characterised in that** zinc oxide gels according to claim 1 are redispersed in organic solvents and/or water, with surface-modifying compounds optionally being added.

7. Process for producing zinc oxide sols according to claim 6, by basic hydrolysis of at least one zinc compound in alcohol or a mixture of alcohol and water, **characterised in that** the precipitate that is initially produced during the hydrolysis is allowed to mature until the zinc oxide has completely flocculated, this precipitate being redispersed by adding dichloromethane and/or chloroform.

8. Use of zinc oxide gels produced by a process according to any one of claims 1 to 5, for the matrix modification of polymers, lacquers and coatings, as an improved vulcanisation activator for rubbers and latices, for the UV protection of polymers, lacquers and coatings, as well as for the UV protection of sensitive organic dyes and pigments.

9. Use of zinc oxide sols produced by a process according to either claim 6 or claim 7, for the matrix modification of polymers, lacquers and coatings, as an improved vulcanisation activator for rubbers and latices, for the UV protection of polymers, lacquers and coatings, as well as for the UV protection of sensitive organic dyes and pigments.

10. Zinc oxide gels, **characterised in that** they have been produced by a process according to any one of claims 1 to 5.

11. Zinc oxide sols, **characterised in that** they have been produced by a process according to either claim 6 or claim 7.

## Revendications

1. Procédé pour la préparation de gels redispersibles à base de nanoparticules d'oxyde de zinc, contenant des particules d'oxyde de zinc avec un diamètre moyen de cercle équivalent de ≤ 15 nm, déterminé par un enregistrement au microscope électronique à transmission, par hydrolyse basique d'au moins un composé de zinc dans l'alcool ou un mélange alcool-eau, **caractérisé en ce que** l'on laisse mûrir le dépôt apparaissant d'abord lors de l'hydrolyse, jusqu'à ce que l'oxyde de zinc soit complètement floculé, on comprime ensuite ce dépôt en un gel et on le sépare de la phase qui le surmonte.

2. Procédé pour la préparation de gels d'oxyde de zinc selon la revendication 1, **caractérisé en ce que** l'on ajoute, avant, pendant ou après la précipitation, 0,01 à 3 % en moles d'ions étrangers, rapportés à la fraction de zinc.

3. Procédé selon une ou plusieurs des revendications 1 et 2, **caractérisé en ce que** l'on utilise comme composé de zinc de l'acétate de zinc et/ou de l'acétate de zinc dihydrate.

4. Procédé selon la revendication 3, **caractérisé en ce que** l'on prépare l'acétate de zinc et/ou l'acétate de zinc dihydrate à partir d'oxyde de zinc au cours d'un procédé préalable.

5. Procédé selon une ou plusieurs des revendications précédentes 1 à 4, **caractérisé en ce que** l'on effectue l'hydrolyse avec des quantités sous-stoechiométriques de base, rapportées au composé de zinc.

6. Procédé pour la préparation de sols d'oxyde de zinc, **caractérisé en ce que** l'on redisperse des gels d'oxyde de zinc selon la revendication 1 dans des solvants organiques et/ou de l'eau, éventuellement avec addition de composés modifiant la surface.

7. Procédé pour la préparation de sols d'oxyde de zinc selon la revendication 6 par hydrolyse basique d'au moins un composé de zinc dans l'alcool ou un mélange alcool-eau, **caractérisé en ce que** l'on laisse mûrir le dépôt apparaissant d'abord lors de l'hydrolyse, jusqu'à ce que l'oxyde de zinc soit complètement floculé et on redisperse ce dépôt par addition de dichlorométhane et/ou de chloroforme.

8. Utilisation de gels d'oxyde de zinc, qui ont été préparés par un procédé selon l'une quelconque des revendications 1 à 5, pour la modification de la matrice de polymères, de laques et de revêtements, comme activateur de vulcanisation amélioré pour des caoutchoucs et des latex, pour la protection UV de polymères, de laques et de revêtements ainsi que pour la protection UV de colorants et de pigments organiques sensibles.

9. Utilisation de sols d'oxyde de zinc, qui ont été préparés par un procédé selon l'une des revendications 6 ou 7, pour la modification de la matrice de polymères, de laques et de revêtements, comme activateur de vulcanisation amélioré pour des caoutchoucs et des latex, pour la protection UV de polymères, de laques et de revêtements ainsi que pour la protection UV de colorants et de pigments organiques sensibles.

10. Gels d'oxyde de zinc, **caractérisés en ce qu'**ils ont été préparés par un procédé selon l'une quelconque des revendications 1 à 5.

11. Sols d'oxyde de zinc, **caractérisés en ce qu'**ils ont été préparés par un procédé selon l'une des revendications 6 ou 7.
